(19) Europäisches Patentamt
European Patent Office
Office européen des brevets

(11) Veröffentlichungsnummer: **0 098 808**
**B1**

(12) # EUROPÄISCHE PATENTSCHRIFT

(45) Veröffentlichungstag der Patentschrift: **26.11.86**

(51) Int. Cl.⁴: **C 09 B 57/00**, C 08 K 5/34, C 07 D 487/04

(21) Anmeldenummer: **83810303.4**

(22) Anmeldetag: **04.07.83**

(54) **Herstellung von 1,4-Diketopyrrolo-(3,4-c)-pyrrolen.**

(30) Priorität: **08.07.82 CH 4171/82**

(43) Veröffentlichungstag der Anmeldung: **18.01.84 Patentblatt 84/3**

(45) Bekanntmachung des Hinweises auf die Patenterteilung: **26.11.86 Patentblatt 86/48**

(84) Benannte Vertragsstaaten: **CH DE FR GB IT LI**

(56) Entgegenhaltungen:
**EP-A-0 061 426**

**TETRAHEDRON LETTERS, Nr. 29, 1974, Seiten 2549-2552, Pergamon Press, Oxford, GB; D.G. FARNUM et al.: "Attempted reformatskii reaction of benzonitrile, 1,4-diketo-3,6-diphenylpyrrolo 3,4-C pyrrole. A lactam analogue of pentalene"**

(73) Patentinhaber: **CIBA-GEIGY AG, Klybeckstrasse 141, CH-4002 Basel (CH)**

(72) Erfinder: **Cassar, Luigi, Dr., Via Orfeo 25, BOlogna (IT)**
Erfinder: **Iqbal, Abul, Dr., Im Schaiengarten 1, CH-4107 Ettingen (CH)**
Erfinder: **Rochat, Alain Claude, Dr., Schiffenenstrasse 38, CH-1700 Fribourg (CH)**

EP 0 098 808 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft ein Verfahren zur Herstellung von 1,4-Diketo-Pyrrolo-[3,4-c]-pyrrolen, die wertvolle Pigmente darstellen. Tetrahedron Lett. 1974, 2549-52, beschreibt ein Verfahren zur Herstellung von 1,4-Diketo-3,6-diphenylpyrrolo-[3,4-c]-pyrrol ausgehend von Benzonitril und Bromessigsäureäthylester in Gegenwart von aktiviertem Zink-Kupfer. Doch sind die bisher erzielten Ausbeuten ungenügend. Wählt man hingegen als Ausgangsstoffe die Verbindungen gemäss nachfolgender Erfindungsdefinition, so erhält man die gewünschten Pyrrolo-[3,4-c]-pyrrole in wesentlich höherer Ausbeute und Reinheit. Ausserdem lassen sich nach dem erfindungsgemässen Verfahren gezielt und einheitlich asymmetrische 1,4-Diketopyrrolo-[3,4-c]-pyrrole herstellen. Dass zudem die erfindungsgemässe Kombination der Ausgangsstoffe zu den gewünschten Pigmenten führt, war umso überraschender, als gemäss Tetrahedron Lett. 1974, 2549-52, die Verbindungen der Formel II dimerisieren sollten und nicht mit den anderen beiden Komponenten reagieren könnten.

Die Erfindung betrifft somit ein Verfahren zur Herstellung von 1,4-Diketopyrrolo-[3,4-c]-pyrrolen der Formel

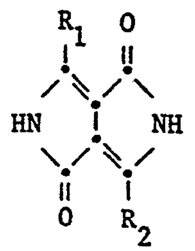

(I)

worin $R_1$ und $R_2$ unabhängig voneinander isocyclische aromatische oder nicht-basische heterocyclische aromatische Reste bedeuten, dadurch gekennzeichnet, dass man 1 Mol der Verbindung der Formel

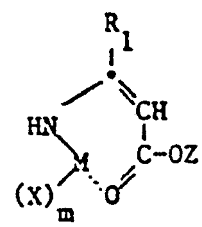

(II)

worin M ein Metallatom, Z Alkyl oder Aryl, X Halogen oder Acetat, und m eine Zahl von Null bis 2 bedeuten, mit 1 Mol einer Verbindung der formel

$YCH_2COOZ$ (III)

worin Y Halogen bedeutet, und mit 1 Mol eines Nitrils der Formel

$R_1$—CN (IV) oder $R_2$—CN (V)

in einem organischen Lösungsmittel bei einer Temperatur von 60 bis 160°C umsetzt und die Verbindung der Formel I isoliert.

Die Reste $R_1$ und $R_2$ können verschieden oder gleich sein. Bedeuten $R_1$ und $R_2$ isocyclische aromatische Reste, dann vorszugsweise monobis tetra-cyclische, insbesondere mono- oder bicyclische Reste, also Phenyl, Diphenylyl oder Naphthyl. Bedeuten $R_1$ und $R_2$ nicht-basische hererocyclische aromatische Reste, dann vorzugsweise mono- bis tricyclische. Diese können rein heterocyclisch sein oder einen heterocyclischen Ring und einen oder mehrere ankondensierre Benzolringe enthalten, wie z.B. ThienyL, Furoyl, Thiophenyl, Cumarinyl oder Benzfuranyl. Sowohl die isocyclischen wie die heterocyclischen aromatischen Reste können die üblichen nicht-wasserlöslichmachenden Substituenten aufweisen, wie:

1) Halogenatome, beispielsweise Chlor, Brom oder Fluor.

2) Verzweigte oder unverzweigte Alkylgruppen mit vorzugsweise 1 bis 18, insbesondere 1 bis 12, vor allem 1 bis 8 und besonders bevorzugt mit 1 bis 4 C-Atomen. Diese Alkylgruppen können nicht-wasserlöslichmachende Substituenten aufweisen, wie beispielsweise Fluor, Hydroxy, Cyano, $-OCOR_3$, $-OR_4$, $-COOR_3$, $-CONR_4R_5$ oder $-R_3-OCONHR_3$, worin $R_3$ Alkyl, Aryl, wie Naphthyl, oder unsubstituiertes oder durch Halogen, Alkyl oder -O-Alkyl substituiertes Benzyl oder einen heterocyclischen Rest, $R_4$ und $R_5$ Wasserstoff, unsubstituiertes oder durch Cyano oder Hydroxy substituiertes Alkyl, $C_5$-$C_6$-Cycloalkyl, Aryl oder Heteroaryl, insbesondere unsubstituiertes oder durch Halogen, Alkyl oder -O-Alkyl substituiertes Phenyl bedeuten, oder worin $R_4$ und $R_5$ zusammen mit dem N-Atom einen 5-6-gliedrigen Heteroring bilden, wie beispielsweise einen Morpholin-, Piperidin- oder Phthalimidring. Weitere mögliche Substituenten an den Alkylgruppen sind mono- oder dialkylierte Aminogruppen, Arylreste, wie Naphthyl oder insbesondere unsubstituiertes oder durch Halogen, Alkyl oder -0-Alkyl substituiertes Phenyl, oder ferner nichtbasische heterocyclische aromatische Reste, wie z.B. die 2-Thienyl-oder 6-Benzimidazolonylreste.

Enthalten die unter 2) genannten Substituenten ihrerseits wieder Alkyl, so kann dieses Alkyl verzweigt oder unverzweigt sein und vorzugsweise l bis 18, insbesondere 1 bis 12, vor allem 1 bis 8 und besonders bevorzugt 1 bis 4 C-Atome enthalten.

Beispiele von unsubstituierten oder substituierten Alkylgruppen sind Methyl, Aethyl, n-Propyl, Isopropyl, n-Butyl, sec.-Butyl, tert.-Butyl, tert.-Amyl, n-Pentyl, n-Hexyl, 1,1,3,3-Tetramethylbutyl, n-Heptyl, n-Octyl, Nonyl, Decyl, Undecyl, Dodecyl, Hydroxymethyl, Trifluormethyl, Trifluoräthyl, Cyammethyl, Methoxycarbonylmethyl, Acetoxymethyl oder Benzyl.

3) Die Gruppe $-OR_6$, worin $R_6$ Wasserstoff, Alkyl, Aryl, beispielsweise Naphthyl oder insbesondere unsubstituiertes oder durch Halogen, Alkyl, oder -0-Alkyl substituiertes Phenyl, $C_5$-$C_6$-Cycloalkyl, Aralkyl oder einen nicht-basischen heterocyclischen Rest bedeutet. In den Definitionen von $R_6$ vorkommendes Alkyl kann die unter 2) für AlkyL als bevorzugt angegebenen Anzahlen von C-Atomen haben. Als Beispiele von $R_6$ seien genannt: Methyl, Aethyl, n-Propyl, Isopropyl, Trifluoräthyl, Phenyl, o-, m- oder p-Chlorphenyl, o-, m- oder p-Methylphenyl, α- oder ß-Naphthyl, Cyclohexyl, Benzyl, Thienyl oder Pyranylmethyl.

4) Die Gruppe $-SR_6$, worin $R_6$ die unter 3) angegebene Bedeutung hat. Als Beispiele für $R_6$ seien genannt: Methyl, Aethyl, n-Propyl, Iso-ProPyl, Phenyl, o-, m- oder p-Chlorphenyl, o-, m- oder p-Methylphenyl, α- oder ß-Naphthyl, Cyclohexyl, Benzyl, Thienyl oder Pyranylmethyl.

5) Die Cyangruppe.

6) Die Gruppe der Formel $-COOR_3$, worin $R_3$ die unter 2) angegebene Bedeutung hat. Als Beispiele für $R_3$ seien genannt: Methyl, Aethyl, Isopropyl, tert.-Butyl, n-Butyl, Phenyl, Benzyl oder Furfuryl.

7) Die Gruppe der Formel $-COR_6$, worin $R_6$ die unter 3) angegebene Bedeutung hat. Als Beispiele seien genannt: Methyl, Aethyl, tert.-Butyl, Phenyl, o-, m- oder p-Chlorphenyl, o-, m- oder p-Methylphenyl oder α- bzw. ß-Naphthyl.

8) Die Gruppe der Formel $-NR_7COR_3$, worin $R_3$ die unter 2) angegebene Bedeutung hat, $R_7$ Wasserstoff, Alkyl, Aryl, beispielsweise Naphthyl oder insbesondere unsubstituiertes oder durch Halogen, Alkyl oder -O-Alkyl substituiertes Phenyl, $C_5$-$C_6$-Cycloalkyl, Aralkyl oder den Rest $-COR_3$ bedeutet, wobei zwei Reste $-COR_3$ zusammen mit dem N-Atom einen heterocyclischen Ring bilden können. In den Definitionen von $R_7$ vorkommendes Alkyl kann z.B. eine der unter 2) als bevorzugt angegebenen Anzahl C-Atome haben. Als Beispiele seien genannt: Acetylamino, Propionylamino, Butyrylamino, Benzoylamino, p-Chlorbenzoylamino, p-Methylbenzoylamino, N-Methylacetylamino, N-Methyl-benzoylamino, N-Succinimido oder N-Phthalimido.

9) Die Gruppe der Formel $-NR_6COOR_3$, worin $R_3$ und $R_6$ die unter 2) bzw. 3) angegebene Bedeutung haben. Als Beispiele seien die Gruppen $-NHCOOCH_3$, $NHCOOC_2H_5$ oder $NHCOOC_6H_5$ genannt.

10) Die Gruppe der Formel $-NR_6CONR_4R_5$, worin $R_6$, $R_5$ und $R_4$ die unter 3) und 2) angegebene Bedeutung haben. Als Beispiele seien genannt: Ureido, N-Methylureido, N-Phenylureido oder N,N-2',4'-Dimethylphenylureido.

11) Die Gruppe der FormeL $-NHSO_2R_3$, worin $R_3$ die unter 2) angegebene Bedeutung hat. Als Beispiele seien genannt: Methansulfonylamino, Phenylsulfonylamino, p-Toluylsulfonylamino oder ß-Naphthylsulfonylamino.

12) Die Gruppen der Formel $-SO_2R_3$ oder $SOR_3$, worin $R_3$ die unter 2) angegebene Bedeutumg hat. Als Beispiele seien genannt: Methylsulfonyl, Aethylsulfonyl, Phenylsulfonyl, 2-NaphthyLsulfonyl, Phenylsulfoxidyl.

13) Die Gruppe der FormeL $-SO_2OR_3$, worin $R_3$ die unter 2) angegebene Bedeutung hat. Als Beispiele für $R_3$ seien genannt: Methyl, AethyL, Phenyl, o-, m- oder p-Chlorphenyl, o-, m- oder p-Methylphenyl, α- oder ß-Naphthyl.

14) Die Gruppe der FormeL $-CONR_4R_5$, worin $R_4$ und $R_5$ die unter 2) angegebene Bedeutung haben. Als Beispiele seien genannt: Carbamoyl, N-Methylcarbamoyl N-Aethylcarbamoyl N-Phenylcarbamoyl, N,N-Dimethyl-carbamoyl, N-Methyl-N-phenylcarbamoyl, N-α-Naphthylcarbamoyl, oder N-Piperidylcarbamoyl.

15) Die Gruppe der Formel $-SO_2NR_4R_5$, worin $R_4$ und $R_5$ die unter 2) angegebene Bedeutung haben. Als Beispiele seien genannt: Sulfamoyl, N-Methylsulfamoyl, N-Aethylsulfamoyl, N-Phenylsulfamoyl, N-Methyl-N-phenylsulfamoyl oder N-Morpholylsulfamoyl.

16) Die Gruppe der Formel $-N=N-R_8$, worin $R_8$ den Rest einer Kupplungskomponente oder einen gegebenenfalls durch Halogen, Alkyl oder -0-Al-kyl substituierten Phenylrest bedeutet. In den Definitionen von $R_8$ vorkommendes Alkyl kann z.B. eine der unter 2) als bevorzugt angegebenen Anzahl C-Atome haben. Als Beispiele für $R_8$ seien genannt: die Acetoacetarylid-, Pyrazolyl-, pyridonyl-, o- oder p-Hydroxyphenyl-, o-Hydroxynaphthyl-, p-Aminophenyl-, oder p-N,N-Dimethylaminophenylreste.

17) Die Gruppe der Formel $-OCOR_3$, worin $R_3$ die unter 2) angegebene Bedeutung hat. Als Beispiele für $R_3$ seien genannt: Methyl, Aethyl, Phenyl, o-, m- oder p-Chlorphenyl.

18) Die Gruppe der Formel $-OCONHR_3$, worin $R_3$ die unter 2) angegebene Bedeutung hat. Als Beispiele für $R_3$ seien genannt: Methyl, Aethyl, Phenyl, o-, m- oder p-Chlorphenyl.

X und Y in den Verbindungen der Formel II bzw. III können als Halogen beispielsweise Chlor, Brom, Jod bedeuten.

Bedeutet Z in den Verbindungen der Formel II und III Alkyl, so kann dieser verzweigt oder unverzweigt sein und vorzugsweise 1 bis 12, vor allem 1 bis 8 und besonders bevorzugt 1 bis 5 C-Atome enthalten. Beispiele für Alkyl sind Methyl, Aethyl, n-Propyl, Isopropyl, n-Butyl, sek.-Butyl, tert.-Butyl, n-Pentyl, tert.-Amyl, n-Hexyl, r Heptyl, n-Octyl und 1,1,3,3-Tetramethylbutyl.

Aryl als Bedeutung von Z in den Verbindungen der Formel II und III bedeutet insbesondere unsubstituiertes oder durch Halogen, wie Chlor, $C_1$-$C_6$-Alkyl, wie Methyl, Aethyl, Isopropyl oder tert.-Butyl, oder $C_1$-$C_6$-Alkoxy, wie Methoxy oder Aethoxy substituiertes Phenyl. Aryl bedeutet bevorzugt unsubstituiertes Phenyl.

M in den Verbindungen der Formel II kann als Metallatom insbesondere ein ein-, zwei- oder dreiwertiges Metallatom bedeuten, wie beispielsweise Lithium, Natrium, Kalium, Magnesium, Calcium, Zink, Kupfer oder Aluminium. Das Metallatom ist bevorzugt Zink. Im erfindungsgemässen Verfahren verwendet man als

Ausgangsstoffe bevorzugt Verbindungen der Formel II, worin $R_1$ unsubstituiertes oder nicht wasserlöslichmachende Substituenten aufweisendes Phenyl oder Naphthyl bedeutet.

Vor allen veiwendet man als Ausgangsstoffe bevorzugt Verbindungen der Formel II, worin $R_1$ den Rest der Formel VI bedeutet,

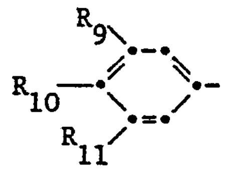

(VI)

worin $R_9$, $R_{10}$ und $R_{11}$ unabhängig voneinander Wasserstoff, Halogen, Carbamoyl, Cyano, Trifluormethyl, $C_2$-$C_{13}$-Alkylcarbamoyl, $C_1$-$C_{12}$-Alkyl, $C_1$-$C_{12}$-Alkoxy, $C_1$-$C_{12}$-Alkylmercapto, $C_2$-$C_{13}$-Alkoxy-carbonyl, $C_2$-$C_{13}$-Alkanoylamino, unsubstituiertes oder durch Halogen, $C_1$-$C_{12}$-Alkyl oder $C_1$-$C_{12}$-Alkoxy substituiertes Phenoxy, Phenylmercapto, Phenoxycarbonyl, Phenylcarbamoyl oder Benzoylamino bedeuten, wobei mindestens einer der Substituenten $R_9$, $R_{10}$ und $R_{11}$ Wasserstoff bedeutet.

Insbesondere verwendet man als Ausgangsstoffe Verbindungen der Formel II, worin $R_1$ der Rest der Formel VII bedeutet

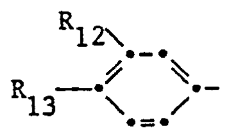

(VII),

worin einer der Substituenten $R_{12}$ und $R_{13}$ Chlor, Brom, $C_1$-$C_4$-Alkyl, Cyano, $C_1$-$C_4$-Alkoxy, unsubstituiertes oder durch Chlor oder Methyl substituiertes Phenoxy, Carbamoyl, $C_2$-$C_5$-Alkylcarbamoyl, unsubstituiertes oder durch Chlor, Methyl oder Methoxy substituiertes phenylcarbamoyl bedeutet, und der andere Wasserstoff ist.

In bevorzugt als Ausgangsstoffe zu verwendenden Verbindungen der Formel II bedeuten X Brom oder Chlor, M Natrium, Kalium, Magnesium oder Zink, m Null oder 1, und Z Alkyl mit 1 bis 8 C-Atomen.

In besonders bevorzugten Verbindungen der Formel II bedeuten X Brom, M Zink, m die Zahl 1,umd Z Alkyl mit 1 bis 5 C-Atomen.

In den Verbindungen der Formel III, die bevorzugt als Ausgangsstoffe verwendet werden,bedeutet Y Brom oder Chlor und Z Alkyl mit 1 bis 8, vor allem 1 bis 5 C-Atomen. Besonders bevorzugt verwendet man Bromessigsäuremethyl- oder -äthylester.

Im erfindungsgemässen Verfahren bevorzugt als Verbindungen der Formelm IV und V verwendete Nitrile sind solche, in denen $R_1$ und $R_2$ unsubstituiertes oder nicht-wasserlöslichmachende Substituenten aufweisendes Phenyl oder Naphthyl bedeuten. Besonders bevorzugt verwendet man Nitrile der Formeln IV und V, worin $R_1$ und $R_2$ Reste der Formel VI, vor allem der Formel VII bedeuten.

Mit Interesse verwendet man als Ausgangsstoffe Verbindungen der Formel II und V, worin $R_1$ und $R_2$ voneinander verschieden sind.

Man führt die Umsetzung der Verbindungen der Formeln II und III mit der Verbindung der Formel IV oder V in einem organischen Lösungsmittel durch. Als Lösungsmittel eignen sich beispielsweise Aether wie Tetrahydrofuran Dioxan, Anisol oder Diphenyläther oder Glykoläther wie Aethylenglykoldimethyläther Aethylenglykoldiäthyläther, Diäthylenglykoldimethyläther, oder Diäthylenglykoldiäthyläther, ferner dipolaraprotische Lösungsmittel, wie Dimethylformamid, N,N-Dimethylacetamid, Nitrobenzol, N-Methylpyrrolidon, aliphatische oder aromatische Kohlenwasserstoffe oder Gemische davon, wie Benzol oder durch Alkyl oder Halogen substituiertes Benzol, vie Toluol, Xylol, Trimethylbenzol, Isopropylbenzol oder Chlorbenzole. Zudem ist es auch möglich, als Lösungsmittel das umzusetzende Nitril der Formel IV bzw. V zu verwenden, falls es im Temperaturbereich flüssig ist, in dem die Umsetzung erfolgt. Die genannten Lösungsmittel können auch als Mischungen eingesetzt werden. Zweckmässigerweise verwendet man 5 bis 20 Gew.-Teile Lösungsmittel auf 1 Gew.-Teil an der Gesamtmenge der Reaktionsteilnehmer.

Im erfindungsgemässen Verfahren verwendet man bevorzugt einen Aether oder einen aromatischen Kohlenwasserstoff als Lösungsmittel, insbesondere Aethylenglykoldimethyläther, Tetrahydrofuran, Dioxan, Anisol, Toluol, o-, m-, p-Xylol, Trimethylbenzol oder Isopropylbenzol.

Das erfindungsgemässe Verfahren wird bei einer Temperatur von 60 bis 160°C, vorzugsweise 90 bis 140°C durchgeführt.

Die Verbindung der Formel 1 kann durch Abfiltrieren isoliert werden. Dabei geht man zweckmässiß so vor, dass man vor dem Filtrieren die Reaktionslösung mit einem organischen Lösungsmittel, wie beispielsweise Aceton, verdünnt, und nach dem Filtrieren die Verbindung der Formel 1 mit Wasser wäscht. Zusätzlich zum Wasser kann man beim Waschen eine anorganische oder organische Säure verwenden, wie beispielsweise

Salzsäure oder Essigsäure.

Bei erfindungsgemässer Herstellung der Verbindungen der Formel I ist es grundsätzlich möglich, bei tieferer Temperatur alle Komponenten vorzulegen und dann das Gemisch in den Bereich der Reaktionstemperatur zu erwärmen, oder in beliebiger Reihenfolge die einzelnen Komponenten im Bereich der Reaktionstemperatur zueinander zuzugeben.

Eine bevorzugte Ausführungsform, die sich in der Regel besonders günstig auf die Ausbeute auswirkt, besteht darin, dass man die Verbindung der Formel II zusammen mit dem aromatischen Nitril der Formel IV oder V vorlegt und die Verbindung der Formel III im Bereich der Reaktionstemperatur zudosiert. Es ist durchaus möglich, das erfindungsgemässe Verfahren nicht nur batchweise, sondern auch kontinuierlich durchzuführen.

Wenn es auch in der Regel genügt, die Reaktanden in stöchiometrischen Mengen einzusetzen, so kann es sich günstig auf die Ausbeute auswirken, wenn man insbesondere die Verbindungen der Foimel IV bzw. V in einem Ueberschuss von bis etwa 2,5 Mol auf 1 Mol der Verbindung der Formel II verwendet.

Das erfindungsgemässe Verfahren erlaubt mit sehr hoher Selektivität die Herstellung von Verbindungen der Formel I, worin $R_1$ und $R_2$ voneinander verschieden sind.

Die Verbindungen der Formel III, IV und V sind bekannt oder lassen sich nach bekannten Verfahren herstellen.

Die Verbindungen der Formel II können hergestellt werden, indem man eine Verbindung der Formel VIII

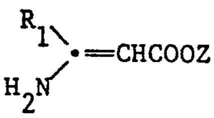

(VIII)

worin $R_1$ und Z die oben angegebene Bedeutung haben, mit einem Metall M oder Metallsalz $MX_m$, wobei M, X und m die oben angegebene Bedeutung haben, unter an sich bekannten Bedingungen umsetzt. Die Verbindungen der Formel VIII ihrerseits lassen sich nach verschiedenen bekannten Verfahren herstellen, beispielsweise durch Umsetzung von Cyanoessigsäureestern mit metallorganischen Verbindungen des Typs $R_1MgX$, wobei X Halogen bedeutet.

Je nach Art ihrer Substituenten und der zu färbenden Polymeren können die Verbindungen der Formel I auch als polymerlösliche Farbstoffe verwendet werden. In der Regel jedoch verwendet man die Verbindungen der Formel I als pigmente für hochmolekulare organische Materialien. Dabei lassen sich die Pigmente im allgemeinen direkt in der Form einsetzen, wie sie nach dem erfindungsgemässen Verfahren anfallen.

Je nach Verwendungszweck kann man die nach dem erfindungsgemässen Verfahren anfallenden Pigmente in eine deckendere oder transparentere Form überführen.

Man kann das Pigment nach der Hydrolyse zuerst isolieren und nachträglich in Wasser oder in einem organischen Lösungsmittel erwärmen, gegebenenfalls unter Druck, um die deckende Form zu erfangen. Vorzugsweise verwendet man solche organische Lösungsmittel, die über 80°C sieden. Als besonders geeignet erweisen sich durch Halogenatome, Alkyl- oder Nitrogruppen substituierte Benzole, wie Xylole, Chlorbenzol, o-Dichlorbenzol oder Nitrobenzol, sowie Pyridinbasen, wie Pyridin, Picolin oder Chinolin, ferner Ketone, wie Cyclohexanon, Aether, wie Aethylenglykolmonomethyl- oder -monoäthyläther, Amide, wie Dimethylformamid oder N-Methylpyrrolidon, sowie Dimethylsulfoxid oder Sulfolan. Man kann die Nachbehandlung auch in Wasser in Gegenwart von organischen Lösungsmitteln und/oder mit Zusatz von oberflächenaktiven Substanzen durchführen.

Hochmolekulare organische Materialien, die mit den Verbindungen der Formel I gefärbt bzw. pigmentiert werden können, sind z.B. Celluloseäther und -ester, wie Aethylcellulose, Nitrocellulose, Celluloseacetat, Cellulosebutylat, natürliche Harze oder Kunstharze, wie Polymerisationsharze oder Kondensationsharze, z.B. Aminoplaste, insbesondere Harnstoff- und Melamin-Formaldehydharze, Alkydharze, Phenoplaste, Polycarbonate, Polyolefine, wie Polystyrol, Polyvinylchlorid, Polyäthylen, Polypropylen, Polyacrylnitril, Polyacrylsäureester, Polyamide, Polyurethane oder Polyester, Gummi, Casein, Silikon und Silikonharze, einzeln oder in Mischungen.

Dabei spielt es keine Rolle, ob die erwähnten hochmolekularen organischen Verbindungen als plastische Massen, Schmelzen oder in Form von Spinnlösungen, Lacken, Anstrichstoffen oder Druckfarben vorliegen. Je nach Veiwendungszweck erweist es sich als vorteilhaft, die erfindungsgemäss zu verwendenden Pigmente als Toner oder in Form von Präparaten einzusetzen. Bezogen auf das zu Pigmentierende hochmolekulare organische Material setzt man die Verbindungen der Formel I in einer Menge von vorzugsweise 0,1 bis 10 Gew.% ein.

Die erhaltenen Färbungen, beispielsweise in Kunststoffen, Fasern, Lacken oder Drucken, zeichnen sich durch grosse Farbstärke, gute Dispergierbarkeit, gute Ueberlackier-, Migrations-, Hitze-, Licht- und Wetterechtheit, sowie durch einen guten Glanz aus.

Die folgenden Beispiele erläutern die Erfindung. Prozente bedeuten Gewichtsprozente.

**Beispiel 1**

Eine Lösung von 9,3 g (0,05 Mol) Allylzinkbromid in 150 ml Tetrahydrofuran wird zu einer Lösung von 9,55 g (0,05 Mol) ß-Aminozimtsäureäthylester in 50 ml Tetrahydrofuran gegeben und während 3 Stunden bei 10°C umgesetzt. Nach dem Abdestillieren des Lösungsmittels unter Vakuum erhält man das Salz der Formel IX

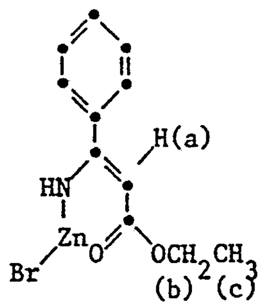

(IX) ,

das mittels $^1$H-NMR in $CD_3CN$ auf einem 250 MHz Bruker N -Spektrophotometer charakterisiert wird und folgende Werte für die chemischen Verschiebungen zeigt: δ (a) 5,14 ppm, δ (b) 4,26 ppm, δ (c) 1,23 ppm.

Das Salz der Formel IX wird in 50 ml Benzonitril gelöst und mit 8,35 g (0,05 Mol) Bromessigsäureäthylester versetzt. Das Gemisch wird 8 Stunden bei 120° C gerührt. Dann kühlt man die rote Suspension ab, versetzt sie mit 50 ml Aceton und rührt weitere 10 Minuten. Nach dem Filtrieren wäscht man den Rückstand mit Aceton, kocht ihn zur Reinigung in 25 ml Eisessig während 4 Stunden bei Rückflusstemperatur, filtriert und wäscht nochmals mit Aceton. Nach dem Trocknen im Vakuum bei 80°C erhält man 3,2 g (22 % der Theorie bezogen auf Bromessigsäureäthylester) reines pigment der Formel X

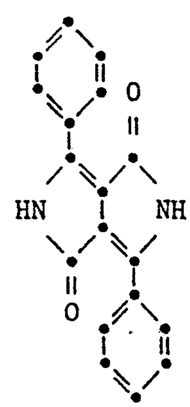

(X) ,

das in PVC eingearbeitet eine rote Färbung ergibt.

<u>Mikroanalyse</u>: $C_{18}H_{12}N_2O_2$ MG 288,3
Ber.: C 74,99 % H 4,22 % N 9,72 %
Gef.: C 74,70 % H 4,40 % N 9,85 %

**Beispiel 2**

0,8 g $Cu(OCOCH_3)_2$-$H_2O$ werden in 25 ml Eisessig bei 90° C gelöst und mit 14,4 g (0,22 Mol) Zinkstaub versetzt. Das Gemisch wird 1 Minute gerührt, dekantiert und mit Eisessig und Benzonitril gewaschen. Das so aktivierte Zink, welches noch 9 ml Benzonitril enthält, wird zu 50 ml (0,5 Mol) Benzonitril gegeben und mit 0,05 g Jod versetzt. Unter Stickstoffatmosphäre tropft man innert etwa 1 Stunde bei 20°C 22,1 ml (0,2 Mol) Bromessigsäureäthylester zu. Nach beendetem Zutropfen wird das Gemisch 12 Stunden bei 20°C gerührt. $^1$H-NMR-Spektroskopie und Gaschromatographie zeigen, dass sich der Bromessigsäureäthylester vollständig umgesetzt hat und 0,2 Mol (67,0 g) des Salzes der Formel IX im Gemisch vorliegen.

Zu diesem Gemisch, das das Salz der Formel IX enthält, werden 100 ml Xylol und 22,1 ml (0,2 Mol) Bromessigsäureäthylester zugegeben. Nach 10 Stunden Rühren bei 130°C wird die rote Suspension abgekühlt, mit 50 ml Aceton versetzt, 10 Minuten gerührt, filtriert und mit Aceton gewaschen. Nach dem Kochen in Eisessig, Abfiltrieren, Waschen mit Aceton und Trocknen erhält man 12,5 g (43 % der Theorie bezogen auf das

Salz der Formel IX) des Pigmentes der Formel X.

Bei der Destillation des Filtrates gewinnt man 45 g Benzonitril und 16 g Bromessigsäureäthylester zurück. Die Ausbeute am Pigment der Formel X beträgt bezogen auf umgesetztes Benzonitril 63 % und bezogen auf umgesetzten Bromessigsäureäthylester 28 %.

**Beispiel 3**

5,1 g 45 %ige Natrium-Dispersion werden in 100 ml Xylol vorgelegt und mit einer Lösung von 17,7 g ß-Aminozimtsäuremethylester in 100 ml Xylol versetzt. Das Gemisch wird auf 60 bis 70° C erwärmt und 30 Minuten bei dieser Temperatur gerührt. Anschliessend wird auf Raumtemperatur abgekühlt. Man gibt 22,5 g Zinkbromid zu und rührt 30 Minuten bei 40°C. Zu dem so in situ hergestellten Zinksalz des ß-Aminozimtsäuremethylesters gibt man eine Mischung aus 11,1 ml Bromessigsäuremethylester und 10,3 ml Benzonitril hinzu. Die resultierende gelbe Suspension wird auf 110°C erwärmt und bei dieser Temperatur 15 Stunden gerührt. Hierauf wird das Reaktionsgemisch mft 40 ml Eisessig versetzt und 15 Minuten bei 100°C gerührt. Nach dem Abkühlen auf 80°C gibt man unter Rühren 200 ml Aceton hinzu, filtriert den ausgefallenen Rückstand ab, wäscht mit Aceton nach und trocknet im Vakuum bei 80°C. Das erhaltene rote Pulver ist gemäss Elementaranalyse identisch mit dem Pigment der Formel X von Beispiel 1.

**Beispiel 4**

Aus 9,3 g (0,05 Mol) Allylzinkbromid und 8,35 g (0,05 Mol) ß-Aminozimtsäuremethylester wird analog Beispiel 1 das Salz der Formel XI hergestellt

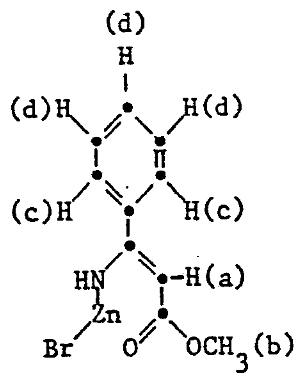

(XI) ,

das mittels $^1$H-NMR-Spektroskopie charakterisiert wird (WM 250 MHz Bruker NMR-Spektrophotometer, $CD_3CN$):

δ (a) 4,88 ppm (s), δ (b) 3,63 ppm (s), δ (c) 7,59 ppm (m), δ (d) 7,46 ppm (m).

Das Salz der Formel XI wird in 30 ml Xylol suspendiert und mit 8,35 g (0,05 Mol) Bromessigsäuremethylester sowie 15,5 g (0,113 Mol) p-Chlorbenzonitril versetzt. Das Gemisch wird während 8 Stunden bei 120°C gerührt. Nach dem Aufarbeiten wie in Beispiel 1 erhält man 4,2 g (26 % der Theorie bezogen auf Bromessigsäuremethylester) des Pigmentes der Formel XII

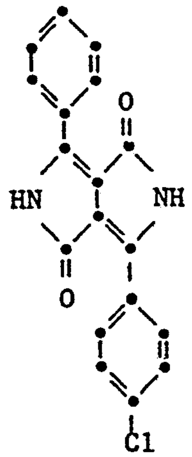

(XII) ,

das in PVC eingearbeitet eine rote Färbung ergibt.
Analyse: $C_{18}H_{11}N_2O_2Cl$ MG 322,5
Ber: C 66,99 % H 3,44 % N 8,68 % Cl 10,99 %
Gef: C 66,50 % H 3,63 % N 8,54 % Cl 10,40 %
$\lambda_{max}$ (nm) gemessen in Dimethylformamid: 471/504

**Beispiele 5 bis 8**

Analog Beispiel 4 erhält man weitere Pigmente der Formel

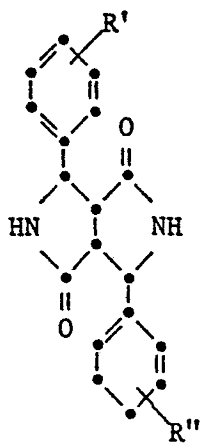

,

worin R′ und R″ die in der folgenden Tabelle aufgeführten Bedeutungen haben.

| Beispiel | R' | R" | Farbe in PVC | $\lambda_{max}$ (nm) gemessen in Dimethylformamid |
|---|---|---|---|---|
| 5 | p-CN | H | rot | 470 / 504 |
| 6 | p-$CH_3$ | H | rot | 470 / 505 |
| 7 | p-$CH_3$ | p-CN | rot | 498 / 520 |
| 8 | p-Cl | p-CN | rot | 488 / 520 |

**Beispiel 9**

7,2 g des gemäss Beispiel 2 aktivierten Zinkes werden in 25 ml Benzonitril aufgeschlämmt und unter Stickstoffatmosphäre mit 8,75 ml Chloressigsäuremethylester über Nacht bei 65°C umgesetzt. $^1$H-NMR-Analyse der Reaktionslösung zeigt eine 40%ige Umsetzung zum Salz der Formel XIII

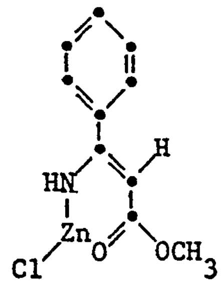

(XIII)

Man gibt 25 ml Xylol und 8,75 ml Chloressigsäuremethylester hinzu und lässt das Gemisch 7 Stunden bei Rückflusstemperatur reagieren. Anschliessend wird analog Beispiel 2 aufgearbeitet. Man erhält 1,4 g (10 % d. Theorie bezogen auf das Salz der Formel XIII des Pigmentes der Formel X.

**Patentansprüche**

1. Verfahren zur Herstellung von 1,4-Diketopyrrolo-[3,4-c]-pyrrolen der Formel

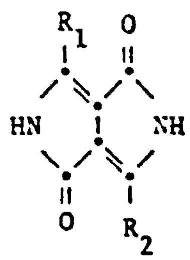

(I)

worin $R_1$ und $R_2$ unabhängig voneinander isocyclische aromatische oder nicht-basische heterocyclische aromatische Reste bedeuten, dadurch gekennzeichnet, dass man 1 Mol der Verbindung der Formel

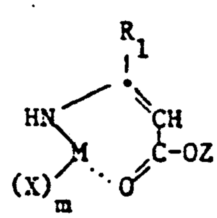

(II)

worin M ein Metallatom, Z Alkyl oder Aryl, X Halogen oder Acetat, und m eine Zahl von Null bis 2 bedeuten, mit 1 Mol einer Verbindung der Formel

$YCH_2COOZ$ (III)

worin Y Halogen bedeutet, und mit 1 Mol eines Nitrils der Formel

$R_1$—CN (IV) oder $R_2$—CN (V)

in einem organischen Lösungsmittel bei einer Temperatur von 60 bis 160°C umsetzt und die Verbindung der Formel I isoliert.

2. Verfahren nach Anspruch 1, dadurch gekennzeichnet, dass man als Ausgangsstoff eine Verbindung der Formel 11 verwendet, vorin $R_1$ unsubstituierte oder nicht-wasserlöslichmachende Substituenten aufweisendes Phenyl oder Naphthyl bedeutet.

3. Verfahren nach Anspruch 1, didurch gekennzeichnet, dass man eine Verbindung der Formel II verwendet, worin $R_1$ den Rest der Formel VI bedeutet,

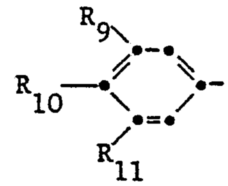

(VI)

worin $R_9$, $R_{10}$ und $R_{11}$ unabhängig voneinander Wasserstoff, Halogen, Carbamoyl, Cyano, Trifluormethyl, $C_2$-$C_{13}$-Alkylcarbamoyl, $C_1$-$C_{12}$-Alkyl, $C_1$-$C_{12}$-Alkoxy, $C_1$-$C_{12}$-Alkylmercapto $C_2$-$C_{13}$-Alkoxycarbonyl $C_2$-$C_{13}$-Alkanoylamino, unsubstituiertes oder durch Halogen, $C_1$-$C_{12}$-Alkyl oder $C_1$-$C_{12}$-Alkoxy substituiertes Phenoxy, Phenylmercapto, Phenoxycarbonyl, Phenylcarbamoyl oder Benzoylamino bedeuten, wobei mindestens einer der Substituenten $R_9$, $R_{10}$ und $R_{11}$ Wasserstoff bedeutet.

4. Verfahren nach Anspruch 1, dadurch gekennzeichnet, dass man eine Verbindung der Formel II veiwendet, worin $R_1$ den Rest der Formel VII bedeutet

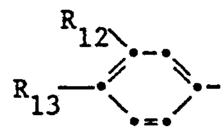

(VII)

worin einer der Substituenten $R_{12}$ und $R_{13}$ Chlor, Brom, $C_1$-$C_4$-Alkyl, Cyano, $C_1$-$C_4$-Alkoxy, unsubstituiertes oder durch Chlor oder Methyl substituiertes Phenoxy, Carbamoyl, $C_2$-$C_5$-Alkylcarbamoyl, unsubstituiertes oder durch Chlor, Methyl oder Methoxy substituiertes Phenylcarbamoyl bedeutet, und der andere Wasserstoff ist.

5. Verfahren nach Anspruch 1, dadurch gekennzeichnet, dass man eine Verbindung der Formel II verwendet, worin X Brom, M Zink, m die Zahl 1 und Z Alkyl mit 1 bis 5 C-Atomen bedeuten.

6. Verfahren nach Anspruch 1, dadurch gekennzeichnet, dass man eine Verbindung der Formel III verwendet, worin Y Brom und Z Alkyl mit 1 bis 5 C-Atomen bedeutet.

7. Verfahren nach Anspruch 1 dadurch gekennzeichnet dass man ein Nitril der Formel IV oder V verwendet, worin $R_1$ und $R_2$ unsubstituierte oder nicht-wasserlöslichmachende Substituenten aufweisendes Phenyl oder Naphthyl bedeuten.

8. Verfahren nach Anspruch 1 und 3, dadurch gekennzeichnet, dass man ein Nitril der Formel IV oder V verwendet, worin $R_1$ und $R_2$ den Rest der Formel VI bedeuten.

9. Verfahren nach Anspruch 1 und 4, dadurch gekennzeichnet, dass man ein Nitril der Formel IV oder V verwendet, vorin $R_1$ und $R_2$ den Rest der Formel VII bedeuten.

10. Verfahren nach Anspruch 1, dadurch gekennzeichnet, dass man als Ausgangsstoffe Verbindungen der Formel II und V vervendet, vorin $R_1$ und $R_2$ voneinander verschieden sind.

11. Verfahren nach Anspruch 1, dadurch gekennzeichnet, dass man als organisches Lösungsmittel einen Aether oder einen aromatischen Kohlenwasserstoff verwendet.

12. Verfahren nach Anspruch 1, dadurch gekennzeichnet, dass man als Lösungsmittel das umzusetzende Nitril der Formel IV bzw. V vervendet.

13. Verwendung der nach Anspruch 1 hergestellten Verbindungen der Formel 1 als Pigmente für hochmolekulares organischs Material.

**Claims**

1. A.process for the preparation of a 1,4-diketopyrrolo-[3,4-c]pyrrole of the formula

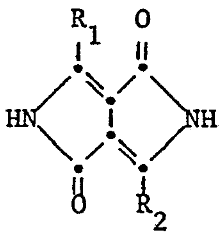

(I),

wherein each of $R_1$ and $R_2$ independently of the other is an isocyclic aromatic or non-basic heterocyclic aromatic radical, which process comprises reacting 1 mole of the compound of formula

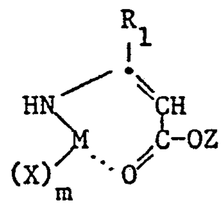

(II)

wherein M is a metal atom, Z is alkyl or aryl, X is halogen or acetate, and m is a value from 0 to 2, with 1 mole of a compound of the formula

$YCH_2COOZ$ (III)

wherein Y is halogen, and with 1 mole of a nitrile of the formula

$R_1$-CN (IV) or $R_2$-CN (V)

in an organic solvent and at a temperature in the range from 60° to 160°C,and isolating the compound of formula I.

2. A process according to claim 1, wherein the starting material is a compound of the formula II, wherein $R_1$ is unsubstituted phenyl or naphthyl or phenyl or naphthyl which carry non-watersolubilising substituents.

3. A process according to claim 1, which comprises the use of a compound of the formula II, wherein $R_1$ is the radical of the formula VI

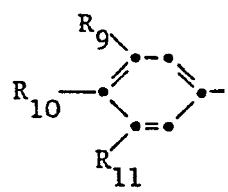

(VI)

wherein each of $R_9$, $R_{10}$ and $R_{11}$ independently is hydrogen, halogen, carbamoyl, cyano, trifluoromethyl, $C_2$-$C_{13}$alkyl-carbamoyl, $C_1$-$C_{12}$alkyl, $C_1$-$C_{12}$alkoxy, $C_1$-$C_{12}$alkylmercapto, $C_2$-$C_{13}$-alkoxycarbonyl, $C_2$-$C_{13}$alkanoylamino, or phenoxy, phenylmercapto, phenoxycarbonyl, phenylcarbamoyl or benzoylamino, each unsubstituted or substituted by halogen, $C_1$-$C_{12}$alkyl or $C_1$-$C_{12}$alkoxy, with the proviso that at least one of $R_9$, $R_{10}$ and $R_{11}$ is hydrogen.

4. A process according to claim 1, which comprises the use of a compund of the formula II, wherein $R_1$ is the radical of the formula VII

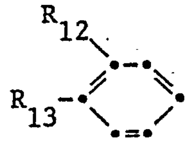

(VII),

wherein one of $R_{12}$ and $R_{13}$ is chlorine, bromine, $C_1$-$C_4$alkyl, cyano, $C_1$-$C_4$alkoxy, or is phenoxy, carbamoyl or $C_2$-$C_5$alkylcarbamoyl, each unsubstituted or substituted by chlorine or methyl, or is phenylcarbamoyl which is unsubstituted or substituted by chlorine, methyl or methoxy, and the other is hydrogen.

5. A process according to claim 1, which comprises the use of a compound of the formula II, wherein X is bromine, M is zinc, m is 1 and Z is $C_1$-$C_5$alkyl.

6. A process according to claim 1, which comprises the use of a compound of the formula III, wherein Y is bromine and Z is $C_1$-$C_5$alkyl.

7. A process according to claim 1, which comprises the use of a nitrile of the formula IV or V, wherein $R_1$ and $R_2$ are unsubstituted phenyl or naphthyl or phenyl or naphthyl which carry non-watersolubilising substituents.

8. A process according to either of claims 1 or 3, which comprises the use of a nitrile of the formula IV or V, wherein $R_1$ and $R_2$ are the radical of the formula VI.

9. A process according to either of claims 1 or 4, which comprises the use of a nitrile of the formula IV or V, wherein $R_1$ and $R_2$ are the radical of the formula VII.

10. A process according to claim 1, wherein the starting materials are compounds of the formulae II and V, wherein $R_1$ and $R_2$ are different.

11. A process according to claim 1, wherein the organic solvent is an ether or an aromatic hydrocarbon.

12. A process according to claim 1, wherein the solvent is the nitrile of the formula IV or V.

13. A method of pigmenting organic material of high molecular weight, which comprises the use of a compound of the formula 1 prepared according to claim 1.

**Revendications**

1. Procédé de préparation de dioxo-1,4 pyrro-10/3,4-c/pyrroles répondant à la formule I:

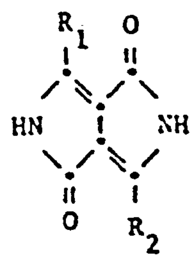

(I)

dans laquelle $R_1$ et $R_2$ représentent chacun, indépendamment l'un de l'autre, un radical aromatique isocyclique ou un radical aromatique hétérocyclique non basique, procédé caractérisé en ce qu'on fait réagir 1 mol d'un composé répondant à la formule II:

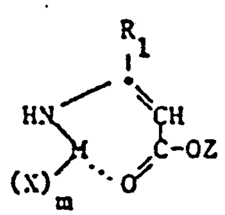

(II)

dans laquelle M représente un atome d'un métal, Z un radical alkyle ou aryle, X un halogène ou un radical acétate et m un nombre de 0 à 2, avec 1 mol d'un composé répondant à la formule III:

$YCH_2COOZ$ (III)

dans laquelle Y représente un halogène, et avec 1 mol d'un nitrile répondant à l'une des formules IV et V:

$R_1$-CN (IV) $R_2$-CN (V)

dans un solvant organique, à une température de 60 à 160°C, et on isole le composé de formule I.

2. Procédé selon la revendication 1 caractérisé en ce qu'on utilise, comme corps de départ, un composé de formule II dans lequel $R_1$ représente un radical phényle ou naphtyle non substitué ou porteur de substituants non hydrosolubilisants.

3. Procédé selon ls revendication 1 caractérisé en ce qu'un utilise un composé de formule II dans lequel $R_1$ représente un radical répondant à la formule VI:

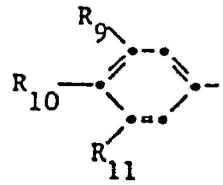

(VI)

dans laquelle $R_9$, $R_{10}$ er $R_{11}$ représentent chacun, indépendamment les uns des autres, l'hydrogène, un halogène, un carbamoyle, un cyano, un trifluorométhyle, un alkylcarbamoyle en $C_2$-$C_{13}$, un alkyle en $C_1$-$C_{12}$, un alcoxy en $C_1$-$C_{12}$, un alkylthio en $C_1$-$C_{12}$, un alcoxycarbonyle en $C_2$-$C_{13}$, un alcanoylamino en $C_2$-$C_{13}$ ou un radical phénoxy, phénylthio, phénoxycarbonyle, phénylcarbamoyle ou benzoylamino non substitué ou porteur d'un halogène, d'un alkyle en $C_1$-$C_{12}$ ou d'un alcoxy en $C_1$-$C_{12}$, au moins l'un des symboles $R_9$, $R_{10}$ et $R_{11}$ représentant l'hydrogène.

4. Procédé selon la revendication 1 caractérisé en ce qu'on utilise un composé de formule II dans lequel $R_1$ représente un radical répondant à la formule VII:

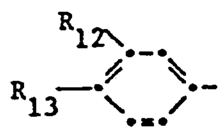

(VII)

dans laquelle l'un des symboles $R_{12}$ et $R_{13}$ représente le chlore, le brome, un alkyle en $C_1$-$C_4$, un cyano, un alcoxy en $C_1$-$C_4$, un phénoxy non substitué ou porteur d'un chlore ou d'un méthyle, un carbamoyle, un alkylcarbamoyle en $C_2$-$C_5$ ou un phénylcarbamoyle non. substitué porteur d'un chlore, d'un méthyl ou d'un méthoxy, et l'autre représente l'hydrogène.

5. Procédé selon la revendicatian 1 caractérisé en ce qu'on utilise un composé de formule II dans lequel X représente le brome, M le zinc, m le nombre 1 et Z un alkyle contenant de 1 à 5 atomes de carbone.

6. Procédé selon la revendication 1 caractérisé en ce qu'on utilise un composé de formule III dans lequel Y représente le brome et Z un alkyle contenant de 1 à 5 atomes de carbone.

7. Procédé selon la revendication 1 caractérisé en ce qu'on utilise un nitrile de formule IV ou V dans lequel $R_1$ et $R_2$ représentent un radical phényle ou naphtyle non substitué ou porteur de substituants non hydrosolubilisants.

8. Procédé selon l'une des revendications 1 et 3, caractérisé en ce qu'on utilise un nitrile de formule IV nu V dans lesquelles $R_1$ et $R_2$ représentent un radical de formule VI.

9. Procédé selon l'une des revendications 1 et 4, caractérisé en ce qu'on utilise un nitrile de formule IV ou V dans lequel $R_1$ et $R_2$ représentent un radical de formule VII.

10. Procédé selon la revendication 1 caractérisé en ce qu'on utilise, comme corps de départ, des composés de formules II et V dans lesquelles $R_1$ et $R_2$ sont différents l'un de l'autre.

11. Procédé selon la revendication 1 caractérisé en ce qu'on utilise, comme solvant organique, un éther ou un hydrocarbure aromatique.

12. Procédé selon la revendication 1 caractérisé en ce qu'on utilise, comme solvant, le nitrile de formule IV ou V à faire réagir.

13. Application des composés de formule 1 préparés selon la revendication 1 comme pigments pour des matières organiques à haut poids moléculaire.